## Europäisches Patentamt

(19) **European Patent Office**

**Office européen des brevets**

(11) Veröffentlichungsnummer: **0 183 739**

**B1**

(12) # EUROPÄISCHE PATENTSCHRIFT

(45) Veröffentlichungstag der Patentschrift:
04.01.89

(21) Anmeldenummer: **85902482.0**

(22) Anmeldetag: **30.05.85**

(86) Internationale Anmeldenummer:
**PCT/DE 85/00193**

(87) Internationale Veröffentlichungsnummer:
**WO 85/05635 (19.12.85 Gazette 85/27)**

(51) Int. Cl.⁴: **C 12 P 17/18 //**
**(C12P17/18, C12R1:645)**

(54) **VERFAHREN ZUR HERSTELLUNG VON -g(a)-ERGOKRYPTIN.**

(30) Priorität: **01.06.84 DE 3420953**

(43) Veröffentlichungstag der Anmeldung:
**11.06.86 Patentblatt 86/24**

(45) Bekanntmachung des Hinweises auf die Patenterteilung:
**04.01.89 Patentblatt 89/1**

(84) Benannte Vertragsstaaten:
**AT BE CH DE FR GB IT LI LU NL SE**

(56) Entgegenhaltungen:
**CH-A-485 020**
**DE-A-3 104 215**
**DE-B-1 617 814**

**Chemical Abstracts, vol. 101, no. 4, 30 July 1984
(Columbus, Ohio, US), p. 411, abstract no. 37214c
Chemical Abstracts, vol. 96, no. 15, 12 April 1982
(Columbus, Ohio, US), Janardhanan K. K. et al.:
"Isolation of ergocryptine from a new strain of
ergot from India", p. 366, abstract 119038r
Chemical Abstracts, vol. 99, no. 11, 11 Sept. 1983
(Columbus, Ohio, US), Sarkisova, M.A. et al.:
"Search for new ergot strains producing peptide
ergoalkaloids", p. 294, abstract 84913e**

(73) Patentinhaber: **SCHERING AKTIENGESELLSCHAFT,
Müllerstrasse 170/178, D-1000 Berlin 65 (DE)**

(72) Erfinder: **WILKE, Detlef, Landwehr 6, D-3015
Wennigsen/Deister (DE)**
Erfinder: **WEBER, Alfred, Schützallee 56, D-1000
Berlin 37 (DE)**

## Beschreibung

Die Erfindung betrifft ein Verfahren zur Herstellung von α-Ergokryptin, welches dadurch gekennzeichnet ist, daß man den Mikroorganismus Claviceps paspali DSM 2836 in einem assimilierbare Kohlenstoff- und Stickstoffquellen und Mineralsalze enthaltenden Nährmedium unter aeroben Kulturbedingungen kultiviert und das gebildete α-Ergokryptin nach Beendigung der Fermentation isoliert.

α-Ergokryptin = 9,10α-Dihydro-12'-hydroxy-2'-(1-methylethyl)-5'α-(2-methylpropyl)-ergotaman-3',6',18-trion ist bekanntlich pharmakologisch wirksam. Nach den bisher bekannten Verfahren wird es aber auf fermentativem Wege nur im Gemisch mit anderen Ergotalkaloiden und Peptidalkaloiden erhalten (Deutsche Offenlegungsschrift 3 104 151). Demzufolge ist seine Herstellung und Isolierung sehr aufwendig.

Es wurde nun gefunden, daß ein Pilzstamm Claviceps spec. α-Ergokryptin in hohen Ausbeuten und neben Spuren von Agroclavin und ansonsten frei von anderen Ergotalkaloiden in das Kulturmedium ausscheidet. Dieser Stamm Claviceps spec. wurde aus einem Mutterkorn eines wild wachsenden Schlickgrases der Species Spartina alterniflora isoliert. Er hat die interne Bezeichnung SCHERING, MBCE 5227 und ist bei der deutschen Sammlung für Mikroorganismen unter der Nummer DSM 2836 hinterlegt. Der Stamm bildet auf Agarmedien mit Saccharose als Kohlenstoffquelle und Asparagin, als Stickstoffquelle flache, gelbliche Kolonien aus kompaktem Mycel. Der Koloniendurchmesser beträgt nach 7 Tagen Kulturzeit 2 bis 3 cm und nach 20 Tagen Kulturzeit 6 bis 8 cm.

Auf Agar-Närmedium mit Saccharose als Kohlenstoffquelle und Ammoniumsuccinat oder Ammoniumcitrat als Stickstoffquelle bildet der Pilz langsam wachsende, gewölbte und von der Agar-Oberfläche leicht abgehobene gelblich braune Kolonien. Die Kolonie hat eine durch Luftmycelbildung rauhe, insidiöse Oberfläche. Der Kolonienrand ist ausgefranst. Der Durchmesser der Kolonie beträgt nach 14 Tagen 2 bis 3 cm und nach 30 Tagen 6 bis 7 cm. Neben hyphenartigen Mycel treten kurze, runde bis unregelmäßig geformte, häufig in Ketten liegende und mit stark lichtbrechenden Vakuolen angefüllte Zellen auf (sklerotialer Zelltyp). Der Hyphendurchmesser beträgt 3 bis 4 um. Die vakuolisierten Zellen haben einen Durchmesser von 6 bis 10 μm und eine Länge von 10 bis 25 μm.

Das erfindungsgemäße Verfahren wird unter Bedingungen durchgeführt, die man üblicherweise zur Anzucht von Pilzkulturen für eine Stoffwechselsynthese anwendet. So werden zunächst in allgemein üblichen Vorversuchen die günstigsten Fermentationsbedinungen, wie z. B. die Auswahl des günstigsten Nährmediums, der technischen Bedingungen wie Temperatur, Belüftung pH-Wert und der optimalen Zeiten für die Germination und die Entwicklung des Mikroorganismus ermittelt.

Als Kohlenstoffquelle kann für das Fermentationsmedium beispielsweise Glucose oder Saccharose verwendet werden. Als Stickstoffquelle dient unter anderem Asparagin, Ammoniumsuccinat oder Ammoniumsulfat. Ferner enthält das Medium die nötigen Wuchsstoffe (beispielsweise Hefeextrakt) und Mineralstoffe (Kalium-, Magnesium-, Kalzium-, Eisen- und Zink-Kationen, sowie Sulfat, Phosphat, Nitrat und Chlorid-Anionen) in der üblicherweise angewendeten Konzentration.

Die Fermentation kann ein- oder zweistufig erfolgen, wobei das für die Vorkultur verwendete Medium mit dem der Hauptkultur identisch sein oder von diesem verschieden sein kann. Für die Vorkultur wird vorzugsweise Glucose als Kohlenstoffquelle verwendet, für die Hauptkultur vorzugsweise Saccharose. Das Vorkulturmedium enthält vorzugsweise 10 bis 100 mg/l Kohlenstoffquellle, das Hauptkulturmedium vorzugsweise 100 bis 300 mg.

Zu Beginn der Fermentation wird der PH-Wert des Mediums vorzugsweise in einem Bereich von 4 bis 6 eingestellt. Die Züchtungstempetatur liegt im Bereich von etwa 10 bis 35°C, vorzugsweise im Bereich von 20 bis 30°C. Die Kulturbedingungen sind streng aerob. Die optimale Fermentationszeit wird durch Analyse des gebildeten α-Ergokryptins in üblicher Weise ermittelt.

Nach erfolgter Fermentation wird das gebildete α-Ergokryptin in an sich bekannter Weise isoliert, beispielsweise indem man die Fermentationsansätze mit einem nicht mit Wasser mischbaren organischen Lösungsmittel, wie Ethylacetat, Metrylisobutylketon, Dichlormethan, Chloroform oder Tetrachlorethan extrahiert, die Extrakte einengt und das erhaltene Rohprodukt durch Chromatographie und/oder Kristallisation reinigt.

Das nachfolgende Ausführungsbeispiel dient zur Erläuterung des erfindungsgemäßen Verfahrens.

## Beispiel

Claviceps spec. DSM 2836 wird auf einem Nährmedium angezüchtet, welches folgende Komponenten enthält:

Saccharose (100 g/l), Citronensäure (10 g/l), Hefeextrakt (0,1 g/l), Kaliumdihydrogenphosphat (500 mg/l), Magnesiumsulfat-Heptahydrat (300 mg/l), Ammoniumsulfat (6 g/l), Calciumnitrat-Tetrahydrat (1 g/l), Eisensulfat-Heptahydrat (7 mg/l), Zinksulfat-Heptahydrat (6 mg/l), Agar (16 g/l). Das Nährmedium ist auf pH 5,1 eingestellt. Die Anzuchtskultur wird 5 bis 20 Tage lang bei 30°C im Brutschrank aufbewahrt.

Ein ca. 1 cm² großes Mycelstück wird mittels eines Ultraturrax unter sterilen Bedingungen in 5 ml physiologischer Kochsalzlösung zerkleinert und damit 50 ml eine Vorkultur enthaltend Glucose (100 g/l), Citronensäure (7,5 g/l),

Kaliumdihydrogenphosphat (0,59 g/l), Magnesiumsulfat-Heptahydrat (300 mg/l), Ammoniumsulfat (6 g/l), Eisen(II)-sulfat-Heptahydrat (7 mg/l), Zinksulfat-Heptahydrat (7 mg/l), Calziumnitrat-Tetrahydrat (1 g/l), Hefeextrakt (0,1 g/l) - eingestellt auf pH 5,1-die sich in einem 500 ml Erlenmeyerkolben befindet, beimpft und auf einem Rundschüttler 4 Tage lang bei 24°C und 220 Umdrehungen pro Minute kultiviert.

5 ml der so erhaltenen Vorkultur werden in 80 ml eines Mediums enthaltend Saccharose (200 g/l), Citronensäure (10 g/l), Hefeextrakt (0,1 g/l), Kaliumdihydrogenphosphat (500 mg/l), Magesiumsulfat-Heptahydrat (300 mg/l), Ammoniumsulfat (6 g/l), Calciumnitrat-Tetrahydrat (1 g/l), Eisensulfat-Heptahydrat (7 mg/l) und Zinksulfat-Heptahydrat (6 mg/l) - eingestellt auf pH 5,1 - die sich in einem 500 ml Erlenmeyerkolben befindet, überführt und 9 Tage lang bei 24°C auf einem Rundschüttler mit 240 Umdrehungen pro Minute geschüttelt.

Dann wird das Kulturmeduim abfiltriert und der Gehalt an α-Ergokryptin mittels Hochdruckflüssigkeitschromatographie (Fresenius Z. Anal. Chem. 303, 1980, 208) ermittelt. Die Konzentration des Kulturfiltrates beträgt 525 mg/l.

**Patentanspruch**

Verfahren zur Herstellung von α-Ergokryptin, dadurch gekennzeichnet, daß man den Mikroorganismus Claviceps paspali DSM 2836 in einem assimilierbare Kohlenstoff- und Stickstoffquellen und Mineralsalze enthaltenden Nährmedium unter aeroben Kulturbedingungen kultiviert und das gebildete α-Ergokryptin nach Beendigung der Fermentation isoliert.

**Claim**

Process for the manufacture of α-ergocryptine, characterized in that the microorganism Claviceps paspali DSM 2836 is cultured in a nutriant medium containing assimilable carbon and nitrogen Sources and mineral salts under aerobic culture conditions, and the α-ergocryptine formed is isolated when fermentation is complete.

**Revendication**

Procédé de préparation de l'α-ergocryptine, caractérisé en ce qu'on cultive en aérobiose le microorganisme claviceps paspali DSM 2836 dans un milieu nutritif contenant des sources de carbone et d'azote assimilables et des sels minéraux, et, après la fin de la fermentation, on isole l'α-ergocryptine formée.